# EUROPÄISCHE PATENTANMELDUNG

(11) **EP 4 846 965 A1**
(43) Veröffentlichungstag der Anmeldung: **02.12.2026**
(21) Anmeldenummer: 26181487.5
(22) Anmeldetag: 28.05.2026
(51) Int. Cl.: A61B 18/14, A61B 17/29, A61B 18/00, A61B 18/12

(54) **BIPOLARES CHIRURGISCHES MAULTEILWERKZEUG UND DAMIT AUSGESTATTETES BIPOLARES CHIRURGISCHES INSTRUMENT SOWIE ROBOTISCHES CHIRURGISCHES INSTRUMENTENSYSTEM**

(30) Priorität: 28.05.2025 DE 102025121164
(71) Anmelder: Karl Storz SE & Co. KG, 78532 Tuttlingen (DE)
(72) Erfinder: Maake, Sagar, 78532 Tuttlingen (DE)

(57) **Zusammenfassung**

Die vorliegende Erfindung stellt ein bipolares chirurgisches Maulteilwerkzeug (8) für ein ebensolches Instrument (100) bereit, ein bipolares chirurgisches Instrument (100) selbst und ein robotisches chirurgisches Instrumentensystem (110). Dabei weist das Maulteilwerkzeug (8) zwei elektrisch leitfähige, voneinander elektrisch isolierte Maulteile (2, 2'), eine Gabelhalterung (5) mit zwei Gabelarmen (51) und ein längliches Betätigungselement auf, das mit den Maulteilen (2, 2') in Eingriff steht. Das Betätigungselement weist distalseitig einen elektrischen Isolatorkörper (1) auf, der zwischen den zwei Gabelarmen (51) geführt wird. Ein erster elektrischer Leitereinsatz (30) und ein zweiter elektrischer Leitereinsatz (40) sind an voneinander abgewandten, den Gabelarmen (51) zugewandten Seiten des Isolatorkörpers (1) befestigt und haben jeweils eine Eingriffsöffnung (31, 41). Jedes Maulteil (2, 2') ist aus einem elektrisch leitenden Material gefertigt und hat an einem Betätigungsabschnitt (20, 20') auf voneinander abgewandten Seiten einen Betätigungszapfen (21, 21') und einen Gelenkzapfen (22, 22'), wobei die Gelenkzapfen (22, 22') beider Maulteile (2, 2') in jeweils einer an den Gabelarmen (51) vorliegenden Schwenköffnung (54) aufgenommen sind. Der Betätigungszapfen (21) des ersten Maulteils (2) ist in der Eingriffsöffnung (31) des ersten Leitereinsatzes (30) aufgenommen, und der Betätigungszapfen (21') des zweiten Maulteils (2') ist in der Eingriffsöffnung (41) des zweiten Leitereinsatzes (40) aufgenommen.

## Beschreibung

Die Erfindung betrifft ein bipolares chirurgisches Maulteilwerkzeug für ein bipolares chirurgisches Instrument und ein bipolares chirurgisches Instrument mit einem solchen Maulteilwerkzeug. Ferner betrifft die Erfindung ein robotisches chirurgisches Instrumentensystem mit einem solchen bipolaren chirurgischen Instrument.

Aus dem Stand der Technik ist bekannt, bipolare chirurgische Instrumente mit einem Maulteilwerkzeug aus beweglichen Maulteilen, wie z. B. Präparier-, Fasszangen und Scheren anzuwenden, die einerseits je nach Form und Größe der Maulteile eine Präparier-, Fass- oder Schneidefunktion und andererseits bei Anlegen von bipolarem Strom eine Koagulationsfunktion haben. Zur Anordnung des Werkzeugs an einem distalen Ende eines mit einem Betätigungsmittel (z. B. Griff oder robotische Handhabe) verbundenen Schafts weist das Instrument einen Werkzeugeinsatz mit einer Maulteilmechanik zum Öffnen und Schließen der Maulteile auf. Die Maulteilmechanik umfasst zumindest ein Gelenk, um dessen Gelenkachse zumindest eine der beiden Maulteile bewegt werden kann. Man unterscheidet zwischen einseitig öffnenden Maulteilwerkzeugen, bei denen nur ein Maulteil bewegt wird, und beidseitig öffnenden Maulteilwerkzeugen, bei denen beide Maulteile bewegt werden. In der Anwendung werden zumeist beidseitig öffnende Maulteilwerkzeuge bevorzugt. Unter Nutzung bipolarer Energie kann Strom kontrolliert zwischen den beiden Maulteile fließen gelassen werden, sodass zwischen den Maulteile befindliches Gewebe koaguliert wird. Da die Maulteile auch als Elektroden fungieren, weisen sie ein elektrisch leitendes Material zur Bildung der Elektroden auf.

Um Kurzschlüsse zwischen den Maulteilen zu vermeiden, bestehen diese üblicherweise nicht vollständig aus dem elektrisch leitenden Material, sondern weisen zumindest im Bereich des Gelenks eine Isolationsschicht auf oder sind mehrteilig mit Isolierabschnitten aufgebaut. Solche Isolatoren aus elektrisch nichtleitenden Kunststoffen oder Keramiken haben meist eine Durchschlagsfestigkeit von 10 bis 50 kV/mm, sodass ein direkter Durchschlag durch den Isolator kaum auftritt, da hier bereits eine Schichtdicke von einem Zehntel Millimeter ausreicht, um mindestens 1.000 V voneinander zu trennen, und mechanische Festigkeit und Fertigung ohnehin meist schon Wandungsstärken von zumindest zwei Zehnteln Millimetern erfordern.

Allerdings ist eine andere Versagensart bei den bipolaren Instrumenten kritisch, nämlich ein Durchschlag entlang der Oberfläche des Isolators entlang einer sogenannten Kriechstrecke. Abhilfe hiergegen ist durch Vergrößerung des geometrischen Abstands zwischen den Maulteilen möglich, um die Kriechstrecke entlang der Isolatoroberfläche möglichst lang zu gestalten, sodass ein Durchschlag erst bei höheren Spannungen stattfindet. Weil aber in der Anwendung leitende Flüssigkeiten (Blut, Saline etc.) den Durchschlag begünstigen und diese sich insbesondere in abgedeckten Bereichen der Maulteilmechanik ansammeln, sind innenliegende Kriechstrecken noch problematischer. Abgesehen von dem Abstand beeinflusst der Werkstoff des Isolators die Anfälligkeit für die Bildung von Kriechstrecken. Hierbei wird die Kriechstromfestigkeit eines Isolatorwerkstoffs mit dem sogenannten CTI-Wert (*Comparative Tracking Index*) bestimmt. Dazu werden 50 Tropfen genormte Elektrolytlösung zwischen zwei Elektroden in vorbestimmtem Abstand auf die Isolatoroberfläche aufgetropft und mit den Elektroden an der Isolatoroberfläche die Spannung gemessen, bis zu der der Isolator kein Tracking zeigt bzw. bei der er leitfähig wird.

Für eine gute Kriechstromfestigkeit sind hohe CTI-Werte des Isolatorwerkstoffs vorteilhaft. Demgegenüber stehen allerdings mechanische Anforderungen an den Werkstoff, weshalb teilweise Kunststoffe als Isolator verwendet werden, die einen niedrigen CTI-Wert von 100 bis 150 aufweisen, wie z. B. PEEK. Denn Kunststoffe mit höherem CTI-Wert sind für die mechanischen Anforderungen häufig zu weich und Keramik ist zu spröde.

Bislang erfüllt keines der bekannten bipolaren Maulteilinstrumente für chirurgische Anwendungen sämtliche daran gestellte Anforderungen, die eine hohe Anwendungsspannung, mechanische Festigkeit, Langlebigkeit, gute Reinigbarkeit und letztendlich geringe Bauteil- und Herstellungskosten umfassen.

Ausgehend von diesem Stand der Technik ist es Aufgabe der vorliegenden Erfindung, ein verbessertes Maulteilwerkzeug für ein bipolares chirurgisches Instrument bereitzustellen.

Diese Aufgabe wird durch ein bipolares chirurgisches Maulteilwerkzeug mit den Merkmalen des Anspruchs 1 gelöst.

Die weitere Aufgabe, ein entsprechend verbessertes bipolares chirurgisches Instrument bereitzustellen, wird durch das bipolare chirurgische Instrument mit den Merkmalen des unabhängigen Anspruchs 13 gelöst.

Die noch weitere Aufgabe, ein entsprechend verbessertes robotisches chirurgisches Instrumentensystem mit einem bipolaren chirurgischen Instrument bereitzustellen, wird durch das robotische chirurgische Instrumentensystem mit den Merkmalen des unabhängigen Anspruchs 14 gelöst.

Weiterbildungen bzw. bevorzugte Ausführungsformen der Vorrichtungen sind in den Unteransprüchen ausgeführt.

Das bipolare chirurgische Maulteilwerkzeug, das dazu ausgebildet ist, mit einem Instrumentenschaft und mit einer Betätigungseinheit ein bipolares chirurgisches Instrument bereitzustellen, weist gemäß einer ersten Ausführungsform zwei elektrisch leitfähige, voneinander elektrisch isolierte Maulteile, eine Gabelhalterung mit zwei Gabelarmen und ein längliches Betätigungselement auf. Die Maulteile sind zwischen den Gabelarmen um jeweils eine Schwenkachse schwenkbar gelagert und stehen mit dem Betätigungselement in Eingriff, das entlang seiner Längsachse relativ zur Gabelhalterung beweglich ist. Das längliche Betätigungselement weist distalseitig einen elektrischen Isolatorkörper auf, der zwischen den zwei Gabelarmen geführt wird. Dabei sind ein erster elektrischer Leitereinsatz und ein zweiter elektrischer Leitereinsatz an voneinander abgewandten, den Gabelarmen zugewandten Seiten des Isolatorkörpers befestigt und weisen jeweils eine Eingriffsöffnung auf. Ferner ist jedes Maulteil aus einem elektrisch leitenden Material gefertigt und hat an einem Betätigungsabschnitt auf voneinander abgewandten Seiten einen Betätigungszapfen und einen Gelenkzapfen. Die Gelenkzapfen beider Maulteile sind in jeweils einer an den Gabelarmen vorliegenden Schwenköffnung aufgenommen und stellen die Schwenkachsen bereit. Der Betätigungszapfen des ersten Maulteils ist in der Eingriffsöffnung des ersten Leitereinsatzes aufgenommen, und der Betätigungszapfen des zweiten Maulteils in der Eingriffsöffnung des zweiten Leitereinsatzes, womit der Eingriff der Maulteile mit dem Betätigungselement bereitgestellt wird.

Bei dem Maulteilwerkzeug sorgt der elektrische Isolatorkörper mit den elektrischen Leitereinsätzen nicht nur für den mechanischen Eingriff der beiden Maulteile mit dem länglichen Betätigungselement, sondern auch für einen sicher voneinander getrennten elektrischen Anschluss der beiden Maulteile.

Unter "elektrischem Isolatorkörper" wird dabei ein Formkörper aus elektrisch isolierendem bzw. nichtleitendem Material verstanden, und "elektrischer Leitereinsatz" meint einen Formeinsatz aus einem elektrisch leitfähigen bzw. leitenden Material, üblicherweise aus einem Metall. Elektrisch leitendes Material, z. B. Metall, weist eine elektrische Leitfähigkeit größer als 10⁵ S/m. Isolatoren bestehen aus einem elektrisch isolierenden Material, z. B. Keramik-, Glas- oder Kunststoff mit einer elektrischen Leitfähigkeit kleiner als 10⁻⁸ S/m.

Durch den Eingriff des Betätigungszapfens in der Eingriffsöffnung werden die Längsbewegung des Betätigungselements auf die Maulteile und gleichzeitig das elektrische Potential übertragen. Der Abstand des Betätigungszapfens von dem Gelenkzapfen bestimmt das Drehmoment zum Drehen des Maulteils um seine Schwenkachse zum Öffnen und Schließen und wird für ein günstiges Hebelarmverhältnis zu dem Funktionsabschnitt des Maulteils möglichst groß gewählt.

Die dabei bereitgestellte Mechanik hat günstige Kraftflüsse, sodass für den Isolatorkörper elektrisch nichtleitenden Materialien mit hohen CTI-Werten verwendet werden können, insbesondere auch Keramiken oder Kunststoffe wie beispielsweise Polybutylenterephthalat (PBT) mit einem CTI von 500, Polyethylen (PE-LD, PE-HD), Polyesterharz, Polytetrafluorethylen (PTFE), Polyvinylidenfluorid (PVDF) jeweils mit einem CTI von 600.

Bezüglich der elektrisch leitenden Materialien und elektrisch isolierenden Materialien der verschiedenen Komponenten des Maulteilwerkzeugs wird folgendes angemerkt: Bei den elektrisch leitenden Materialien der elektrisch angeschlossenen Komponenten wie den vorgenannten Maulteile und Leitereinsätze sowie sämtliche nachfolgend angeführte elektrisch leitenden Komponenten kann es sich um dasselbe elektrisch leitende Material oder um unterschiedliche elektrisch leitende Materialien handeln, die der Fachmann nach den jeweiligen Anforderungen entsprechend auszuwählen weiß. Entsprechendes gilt in ähnlicher Weise für die elektrisch isolierenden Materialien des vorgenannten Isolatorkörpers und sämtlicher nachfolgend angeführter elektrisch isolierender Komponenten. D. h., die elektrisch isolierender Komponenten des Maulteilwerkzeugs können das gleiche elektrisch isolierende Material aufweisen, oder es können je nach Anforderung an die jeweilige Komponente unterschiedliche elektrisch isolierende Materialien verwendet werden.

Nach einer weiteren Ausführungsform des Maulteilwerkzeugs weist das längliche Betätigungselement ein mit dem Isolatorkörper verbundenes Kraftübertragungselement auf, das ein elektrisch leitfähiges inneres Stangenelement und ein elektrisch leitfähiges äußeres Rohrelement umfasst. Das äußere Rohrelement ist koaxial um das innere Stangenelement angeordnet und durch eine innere Isolierhülle von dem inneren Stangenelement elektrisch isoliert. Dabei ist das innere Stangenelement mit dem ersten Leitereinsatz elektrisch verbunden, und das äußere Rohrelement ist mit dem zweiten Leitereinsatz elektrisch verbunden. Beispielsweise kann die elektrische Verbindung der Leitereinsätze mit dem jeweiligen Stangen- und Rohrelement durch eine Schweißverbindung sichergestellt werden.

Das "längliche Betätigungsmittel", das den Isolatorkörper als distales Endstück an diesem Kraftübertragungselement aus innerem Stangen- und äußerem -Rohrelement aufweist, ist somit eine langestreckte Vorrichtung, die eine an der proximalen Betätigungseinheit eines chirurgischen Instruments erzeugte, translatorische Kraft bzw. Bewegung auf die distale Maulteilmechanik überträgt. Beispiele für das Kraftübertragungselement umfassen starre oder flexible Rohranordnungen, wobei sich die Längsachse eines flexiblen Kraftübertragungselements auf seine gestreckte Anordnung bezieht. Richtungsangaben, die auf die Längsachse bezogen sind, gelten für flexible Kraftübertragungselemente dabei nicht nur für die gestreckte Anordnung, sondern auch in entsprechend abgewandelter Weise, bei gebogener Anordnung des flexiblen Kraftübertragungselements. Z. B. sind Bewegungen des länglichen Betätigungsmittels entlang der Längsachse allgemeiner als Hin- und Herbewegungen innerhalb des Instrumentenschafts zu verstehen, also auch bei gebogenem Verlauf.

Die "innere Isolierhülle" ist eine Schicht aus elektrisch isolierendem Material, die das innere Stangenelement umhüllt und einen Kurzschluss oder Durchschlag zwischen dem inneren Stangenelement und dem äußeren Rohrelement zu verhindern, das vorzugsweise ebenfalls von einer Schicht aus elektrisch isolierendem Material, d. h. einer äußeren Isolierhülle, umgeben sein kann.

Nach einer weiteren Ausführungsform des Maulteilwerkzeugs sind der erste Leitereinsatz und der zweite Leitereinsatz durch einen Klebstoff an dem Isolatorkörper befestigt. Alternativ können der erste Leitereinsatz und der zweite Leitereinsatz durch Löten oder Schweißen an dem Isolatorkörper befestigt sein.

Nach noch einer weiteren Ausführungsform des Maulteilwerkzeugs ist vorgesehen, dass der Isolatorkörper auf den voneinander abgewandten Seiten mit einer erste Aufnahmeausnehmung und einer zweiten Aufnahmeausnehmung geformt ist. Die beiden Aufnahmeausnehmungen auf den voneinander abgewandten Seiten sind zur schwenkbaren Führung des Betätigungsabschnitts der Maulteile ausgebildet. Dabei weist die erste Aufnahmeausnehmung eine erste Aufnahmetasche auf, die zur Aufnahme des ersten Leitereinsatzes ausgebildet ist. Entsprechend weist die zweite Aufnahmeausnehmung eine zweite Aufnahmetasche auf, die zur Aufnahme des zweiten Leitereinsatzes ausgebildet ist.

Vorteilhaft wird die Befestigung der Leitereinsätze an dem Isolatorkörper durch die Aufnahmetaschen gesichert, die entsprechend den Leitereinsätzen geformt sind. Die Sicherung wird durch Formschluss erreicht, wobei die bündige Anordnung der Leitereinsätze die stabile Führung der jeweiligen Betätigungsabschnitte nicht beeinträchtigt.

Nach noch einer weiteren Ausführungsform des Maulteilwerkzeugs kann der Isolatorkörper auf den voneinander abgewandten Seiten proximalseitig mit seitlichen Führungsflächen ausgebildet sein, deren Abstand einem Abstand zwischen den Gabelarmen entspricht. Damit wird die Bewegung des Isolatorkörpers mit dem Betätigungselements zum Öffnen und Schließen der Maulteile entlang der Längsachse zwischen den Gabelarmen geführt.

Ferner ist zur Verbindung des Isolatorkörpers und des Kraftübertragungselements nach einer weiteren Ausführungsform des Maulteilwerkzeugs vorgesehen, dass der Isolatorkörper proximalseitig entlang der Längsachse eine Aufnahmebohrung aufweist, in die sich das Kraftübertragungselement erstreckt. D. h. anders ausgedrückt, dass ein distaler Endabschnitt des inneren Stangeelements und des äußeren Rohrelements in der Aufnahmebohrung angeordnet ist. Ferner weist der Isolatorkörper einen ersten Leiterkanal und einen zweiten Leiterkanal auf, die sich von der Aufnahmebohrung zu den voneinander abgewandten Seiten des Isolatorkörpers erstrecken, an denen die beiden Leitereinsätze befestigt sind. Dabei weist der erste Leitereinsatz einen Leiterabschnitt auf, der sich durch den ersten Leiterkanal zur Kontaktierung des inneren Stangenelements erstreckt. Und der zweite Leitereinsatz hat einen Leiterabschnitt, der sich durch den zweiten Leiterkanal zur Kontaktierung des äußeren Rohrelements erstreckt.

Gemäß einer noch weiteren Ausführungsform des Maulteilwerkzeugs liegt eine Verbindungsstelle des ersten Leiterkanals mit der Aufnahmebohrung distal zu einer Verbindungstelle des zweiten Leiterkanals mit der Aufnahmebohrung. Ferner ragt das innere Stangenelement mit der inneren Isolierhülle distal aus dem äußeren Rohrelement hinaus, wobei die innere Isolierhülle einen distalen Endabschnitt des inneren Stangenelements frei lässt, und ein distaler Endabschnitt der Isolierhülle, der sich distal des äußeren Rohrelements erstreckt, einen Kriechstreckenabschnitt bereitstellt.

Als "Kriechstreckenabschnitt" wird vorliegend der aus dem äußeren Rohrelement herausragende distale Endabschnitt der inneren Isolierhülle bezeichnet, der dafür sorgt, dass zwischen den distalen Endabschnitten des inneren Stangenelements und des äußeren Rohrelements kein Durchschlag stattfindet. Dazu wird die Länge des Kriechstreckenabschnitts auf das elektrisch isolierende Material der inneren Isolierhülle abgestimmt, um einen Durchschlag entlang der Oberfläche der Isolierhülle zu vermeiden. Dabei kann der Kriechstreckenabschnitt umso kürzer ausfallen, je höher der CTI-Wert des elektrisch isolierenden Materials der inneren Isolierhülle ist. Der Kriechstreckenabschnitt der inneren Isolierhülle trennt damit die durch das inneren Stangenelement und das äußere Rohrelement bereitgestellten elektrischen Pole in Längsrichtung, wobei die Verbindungsstellen der Leiterkanäle entsprechend in Bezug auf die Längsachse voneinander beabstandet sind. Und da die Eingriffsöffnungen der Leitereinsätze rotationssymmetrisch in Bezug auf die Längsachse am Isolatorkörper angeordnet sind, sind Leiterabschnitte, die sich von den Leitereinsätzen zu den jeweiligen Verbindungsstellen erstrecken, unterschiedlich lang. Daher ist der Leiterabschnitt des ersten Leitereinsatzes, der das inneren Stangenelement kontaktiert, kürzer als der Leiterabschnitt des zweiten Leitereinsatzes, der das äußere Rohrelement kontaktiert, das proximal zu dem inneren Stangenelement in der Aufnahmebohrung endet.

Nach noch einer weiteren Ausführungsform des Maulteilwerkzeugs kann dieses zur Verbindung des Isolatorkörpers mit dem Kraftübertragungselement ein Arretierelement aufweisen, das in einer Arretieröffnung des Isolatorkörpers angeordnet ist, die sich radial zu der Aufnahmebohrung erstreckt.

Das Arretierelement sichert die Verbindung des Isolatorkörpers mit dem Kraftübertragungselement in Längsrichtung durch Aufbringung einer Haltekraft in Querrichtung, damit der Isolatorkörper ohne Spiel oder Schlupf mit dem Kraftübertragungselement hin- und herbewegt wird. Als Arretierelement kommen daher vorzugsweise Verbindungselemente wie Bolzen, Stifte oder Schrauben in Frage, die üblicherweise zylinderförmig sind.

Gemäß einer noch weiteren Ausführungsform des Maulteilwerkzeugs ist die Arretieröffnung in einem Bereich der Aufnahmebohrung ausgebildet, der sich von der Verbindungsstelle des ersten Leiterkanals zu der Verbindungstelle des zweiten Leiterkanals erstreckt. Dann kontaktiert das in der Arretieröffnung angeordnete Arretierelement das distal aus dem äußeren Rohrelement hinausragende innere Stangenelement mit der Isolierhülle an dem Kriechstreckenabschnitt.

Eine noch weitere Ausführungsform des Maulteilwerkzeugs betrifft die Schwenköffnung, in der der Gelenkzapfen des jeweiligen Maulteils gelagert ist. Die Schwenköffnung ist in einem Gabelschalenelement ausgebildet, das in einer an den Gabelarmen ausgebildeten Aussparung angeordnet wird. Dabei sind zumindest die Gabelschalenelemente oder die Gabelarme der Gabelhalterung oder vorzugsweise die Gabelschalenelemente und die Gabelarme aus einem elektrisch isolierenden Material gefertigt
Weitere Ausführungsformen des Maulteilwerkzeugs beziehen sich darauf, dass jedes Maulteil einteilig aus dem elektrisch leitenden Material gefertigt ist, und / oder dass der Betätigungsabschnitt zum flächigen Kontakt mit dem jeweiligen Leitereinsatz ausgebildet ist, wenn der Betätigungszapfen des jeweiligen Maulteils in der Eingriffsöffnung des jeweiligen Leitereinsatzes aufgenommen ist.

Ein bipolares chirurgisches Instrument als weiterer erfindungsgemäßer Gegenstand weist eine Betätigungseinheit, einen Instrumentenschaft und ein Maulteilwerkzeug mit zwei in einer Gabelhalterung schwenkbar gelagerten Maulteile und einem länglichen Betätigungselement auf. Die elektrisch leitfähigen, voneinander elektrisch isolierten Maulteile sind mit der Gabelhalterung an einem distalen Ende des Instrumentenschafts angeordnet. Das längliche Betätigungselement erstreckt sich entlang der Längsachse beweglich durch den Instrumentenschaft und ist mit der Betätigungseinheit gekoppelt, die an einem proximalen Ende des Instrumentenschafts angeordnet ist. Vorteilhaft ist das Maulteilwerkzeug des bipolaren chirurgischen Instruments ein erfindungsgemäßes bipolares chirurgisches Maulteilwerkzeug.

In einer Ausführungsform des bipolaren chirurgischen Instruments kann die Betätigungseinheit des bipolaren chirurgischen Instruments ein händisch betätigbares Griffstück sein, und in einer anderen Ausführungsform eine robotisch betätigbare Handhabe.

Ein weiterer erfindungsgemäßer Gegenstand ist ein robotisches chirurgisches Instrumentensystem, das zumindest eine Steuerungseinheit, einen elektrochirurgischen Generator und einen Roboterarm aufweist, der mit der Steuerungseinheit verbunden ist und ein erfindungsgemäßes bipolares chirurgisches Instrument aufweist, das mit dem elektrochirurgischen Generator verbunden ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung. Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Dabei zeigen:
- **Fig. 1**: eine perspektivische Ansicht eines Maulteilwerkzeugs mit geöffneten Maulteilen,
- **Fig. 2**: eine perspektivische, teilweise Explosionsdarstellung des Maulteilwerkzeugs aus Fig. 1 mit geschlossenen Maulteilen,
- **Fig. 3**: eine Seitenansicht eines bipolaren chirurgischen Instruments,
- **Fig. 4**: eine perspektivische Ansicht eines robotischen chirurgischen Instrumentensystems mit einem bipolaren chirurgischen Instrument,
- **Fig. 5**: eine perspektivische Ansicht des Isolatorkörpers eines Maulteilwerkzeugs von einer ersten Seite,
- **Fig. 6**: eine perspektivische Ansicht des Isolatorkörpers aus Fig. 5 von einer anderen Seite,
- **Fig. 7**: eine perspektivische Ansicht des Isolatorkörpers aus Fig. 5 mit einem ersten Leitereinsatz vor Anordnung eines ersten Maulteils,
- **Fig. 8**: eine weitere perspektivische Ansicht des Isolatorkörpers aus Fig. 7 mit angeordnetem ersten Maulteil,
- **Fig. 9**: eine perspektivische Ansicht des Isolatorkörpers aus Fig. 8 mit einem zweiten Leitereinsatz vor Anordnung des zweiten Maulteils,
- **Fig. 10**: eine perspektivische Ansicht des Isolatorkörpers aus Fig. 9 mit angeordnetem zweiten Maulteil,
- **Fig. 11**: eine weitere perspektivische Ansicht des Maulteilwerkzeugs aus Fig. 1 mit geschlossenen Maulteilen,
- **Fig. 12**: eine weitere perspektivische Ansicht des Maulteilwerkzeugs aus Fig. 1 mit geöffneten Maulteilen.

Die Erfindung betrifft ein Maulteilwerkzeug für bipolare chirurgische Instrumente mit zwei gegeneinander bewegbaren Maulteile wie beidseitig öffnende Zangen- und Scheren-Instrumente. Ferner betrifft die Erfindung ein mit einem entsprechenden Maulteilwerkzeug ausgestattetes bipolares chirurgisches Instrument. Die Maulteilmechanik mit dem speziellen, bifunktionellen Isolatorkörper aus elektrisch isolierendem, d. h. nichtleitenden, Material dient gleichzeitig zur elektrischen Trennung der Maulteile und zur Übertragung einer Betätigungskraft auf die Maulteile. Damit sind keine zusätzlichen Isolatorkomponenten an den Maulteilen nötig, die daher einstückig aus einem elektrisch leitfähigen Material gefertigt sein können.

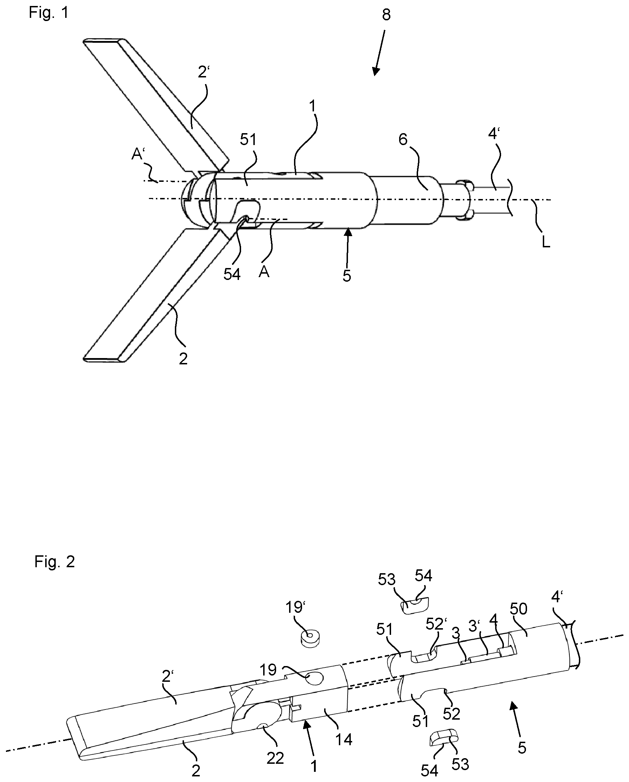

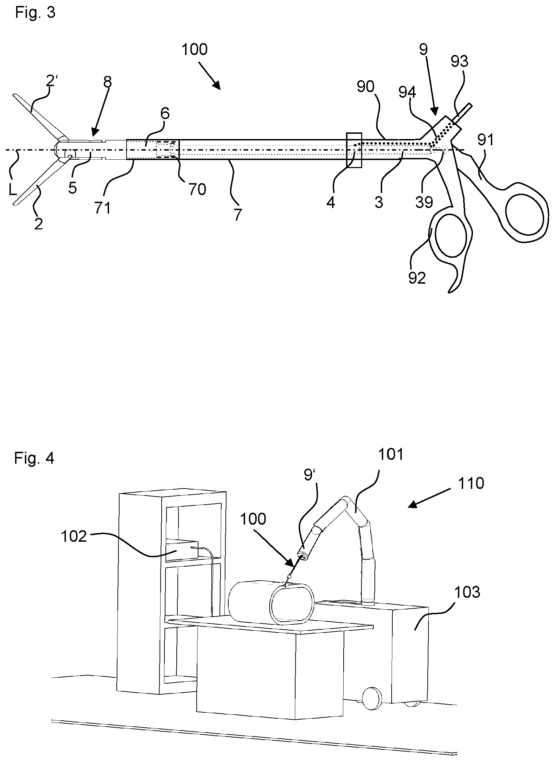

Das in den Figuren 1 bis 12 dargestellte Maulteilwerkzeug 8 ist als Werkzeugeinsatz für ein bipolares chirurgisches Instrument 100 ausgebildet, wie in Fig. 3 gezeigt ist. Die beiden als Bipolarelektroden dienenden Maulteile 2, 2' des Maulteilwerkzeugs 8 sind in einer Gabelhalterung 5 schwenkbar gelagert, die an dem distalen Ende eines Instrumentenschafts 7 angeordnet ist. Die Gabelhalterung 5 hat einen zylindrischen Rohrabschnitt 50, von dem sich zwei Gabelarme 51 distal erstrecken. Die Gabelarme 51 haben einen kreisabschnittsförmigen Querschnitt, sodass ihre Innenseiten parallele Flächen bilden und die Außenseiten die Zylinderform des Rohrabschnitts 50 fortsetzen. Ein längliches Betätigungsmittel des Maulteilwerkzeugs 8 mit einem inneren Stangenelement 3 und einem koaxialen äußeren Rohrelement 4 erstreckt sich durch den Rohrabschnitt 50 der Gabelhalterung 5 und steht mit den Maulteilen 2, 2' in Wirkverbindung.

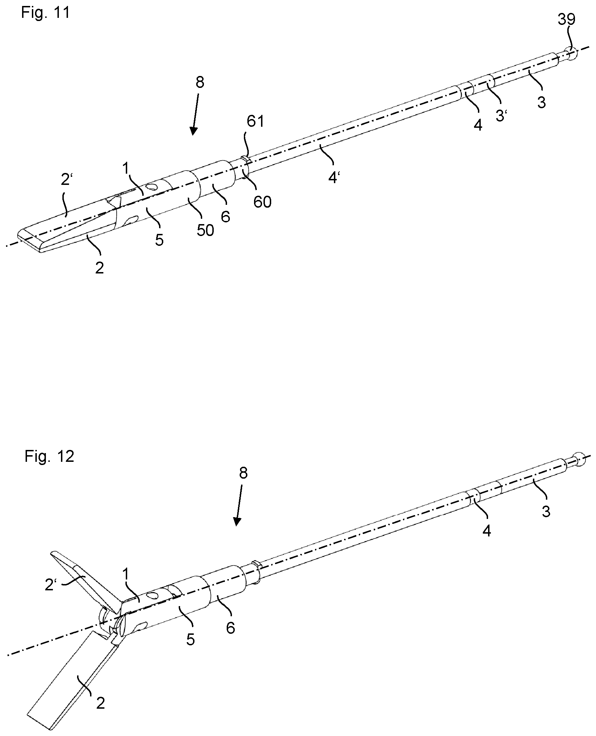

Zur Verbindung der Gabelhalterung 5 mit dem Instrumentenschaft 7 weist das dargestellte Maulteilwerkzeug 8 eine Schaftverbinderhülse 6 auf, die mit dem Rohrabschnitt 50 der Gabelhalterung 5 drehfest verbunden ist. An einem proximalseitigen Verbindungsabschnitt 60 mit verringertem Durchmesser (vgl. Fig. 11) weist die Schaftverbinderhülse 6 hier zwei Bajonettnocken 61 als Schaftverbindungselemente auf, die mit entsprechenden Bajonettnuten (nicht abgebildet) an einem distalen Verbindungsabschnitt 70 des Instrumentenschafts 7 (vgl. Fig. 3) in Eingriff treten können. Dazu alternative Verbindungselemente zur (lösbaren) Verbindung eines Maulteilwerkzeugs 8 mittels einer Schaftverbinderhülse 6 an einem Instrumentenschaft 7 umfassen z. B. Schraub-, Steck- und Rastverbindungen. Bei dem in Fig. 3 dargestellten Instrument 100 ist ferner zur Sicherung der Bajonettverbindung und zur Ebnung der Außenkontur zwischen der Gabelhalterung 5 und dem Instrumentenschaft 7 eine Sicherungshülse 71 vorgesehen, die die Schaftverbinderhülse 6 und distalen Verbindungsabschnitt 70 überdeckt, deren Außendurchmesser kleiner ist als der Außendurchmesser der Gabelhalterung 5 und des Instrumentenschafts 7.

Ferner erstrecken sich das innere Stangen- und äußere Rohrelement 3, 4 entlang einer gemeinsamen Längsachse L längsbeweglich durch den Instrumentenschaft 7, der an seinem proximalen Ende mit einer Betätigungseinheit 9 verbunden ist. Die Betätigungseinheit 9 ist zum Eingriff mit dem länglichen Betätigungsmittel ausgebildet, das dazu im dargestellten Beispiel ein kugelförmiges Kopplungselement 39 am proximalen Ende des inneren Stangenelements 3 aufweist. Selbstverständlich ist es möglich, dass ein Maulteilwerkzeug dazu alternative proximale Kopplungselemente zum Eingriff mit einer Betätigungseinheit aufweist, um das längliche Betätigungselement zum Öffnen und Schließen der Maulteile 2, 2' entlang der Längsachse L hin und her zu bewegen.

Fig. 3 zeigt als Betätigungseinheit 9 ein händisch betätigbares Griffstück 9 mit einem Griffgehäuse 90, das mit dem Instrumentenschaft 7 verbunden ist und das proximale Ende des länglichen Betätigungselements aufnimmt. Ein relativ zu einem starren Griffteil 92 bewegliches Griffteil 91, das an dem Griffgehäuse 90 schwenkbar gelagert ist, steht mit dem proximalen Kopplungselement 39 des länglichen Betätigungselements zum Öffnen und Schließen der Maulteile 2, 2' in Eingriff. Ferner ist das Griffgehäuse 90 mit einer Anschlussbuchse 93 ausgestattet, um das bipolare chirurgische Instrument 100 zur Versorgung mit elektrischer Energie an einem Generator (in Fig. 3 nicht dargestellt) anzuschließen. Durch den Griff 9 wird eine bipolare elektrische Verbindung der Anschlussbuchse 93 mit dem inneren Stangenelement 3 und dem äußeren Rohrelement 4 durch elektrische Anschlusselemente 94 hergestellt, die durch die gepunkteten Linien angedeutet sind. Dazu ragt inneren Stangenelement 3 proximal über das äußere Rohrelement 4 hinaus. Wie auch in Fig. 11 zu erkennen ist, ist dabei das innere Stangenelement 3 von einer inneren Isolierhülle 3' und das äußere Rohrelement 4 von einer äußeren Isolierhülle 4' umgeben, um die Pole zum bipolaren Anschluss der Maulteile 2, 2' zu trennen, wobei jeweils ein proximaler Endabschnitt des inneren Stangenelements 3 und des äußeren Rohrelements 4 unisoliert bleibt, um das jeweilige elektrische Anschlusselement 94 im Griffstück 9 zu kontaktieren.

Alternativ zu einem händisch betätigbaren Griffstück 9 ist es auch möglich, dass ein bipolares chirurgisches Instrument 100 eine robotische Betätigungseinheit 9' aufweist, die mittels eines Eingabegeräts gesteuert wird. Fig. 4 zeigt ein robotisches chirurgisches Instrumentensystem 110 mit einem bipolaren chirurgischen Instrument 100. Dieses befindet sich an einem Ende eines Roboterarms 101, der die Betätigungseinheit 9' des bipolaren chirurgischen Instruments 100 bereitstellt. Der Roboterarm 101 ist mit einer Steuerungseinheit 102 verbunden und stellt die elektrische Verbindung zwischen dem elektrochirurgischen Generator 103 und dem bipolaren chirurgisches Instrument 100 bereit.

In jedem Fall sind die beiden Maulteile 2, 2' des Maulteilwerkzeugs 8 an einem Isolatorkörper 1 gelagert, der aus einem elektrisch isolierenden Material besteht. Hierfür wird vorzugsweise ein Kunststoff- oder Keramikmaterial mit hohem CTI-Wert gewählt. Beispielsweise kann der Isolatorkörper 1 aus einem Kunststoff mit hohem CTI-Wert wie Polyvinylidenfluorid PVDF (CTI-Wert 600) gefertigt, z. B. gefräst oder in Serie im Spritzguss hergestellt werden. Vorteilhaft ist es aber auch möglich, den Isolatorkörper 1 in Serie aus Keramik herzustellen, und dadurch beste mechanische, chemische und elektrische Eigenschaften zu erhalten.

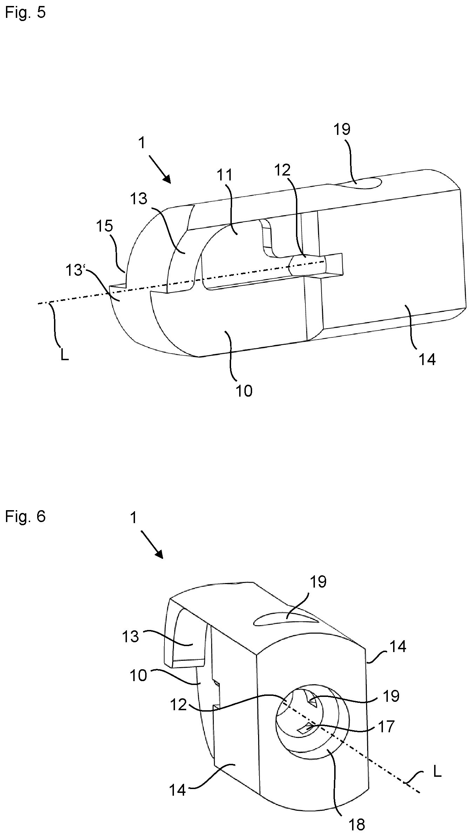

Der in Fig. 5 und 6 dargestellte Isolatorkörper 1 bildet die distale Spitze des länglichen Betätigungselements an einem Kraftübertragungselement, das aus dem inneren Stangenelement 3 und dem äußeren Rohrelement 4 mit den jeweiligen Isolierhüllen 3', 4' gebildet wird. Zum Eingriff mit den Maulteilen 2, 2' ist der Isolatorkörper 1 zwischen den Gabelarmen 51 der Gabelhalterung 5 angeordnet und wird dabei bei der Hin- und Herbewegung des länglichen Betätigungselements entlang der Längsachse L geführt. Dazu weist der Isolatorkörper 1 proximalseitig auf voneinander abgewandten, den Gabelarmen 51 zugewandten Seiten zwei parallele seitliche Führungsflächen 14 auf, deren Abstand einem Abstand zwischen den parallelen Innenseiten der Gabelarmen 51 entspricht, wie in Fig. 2 entnehmbar ist.

Fig. 11 und 12 zeigen, dass sich der Isolatorkörper 1 zusammen mit dem Kraftübertragungselement aus dem inneren Stangen- und dem äußeren Rohrelement 3, 4 mit den jeweiligen Isolierhüllen 3', 4' zum Öffnen und Schließen der Maulteile 2, 2' entlang der Längsachse L hin und her bewegt. Im geschlossenen Zustand der Maulteile 2, 2' gibt es keinen Spalt zwischen dem Isolatorkörper 1 und dem Rohrabschnitt 50 der Gabelhalterung 5 (Fig. 11). Und wenn zum Öffnen der Maulteile 2, 2' das Kraftübertragungselement in distale Richtung verschoben wird, wird der Isolatorkörper 1, der mit den Maulteilen 2, 2' in Eingriff steht, auch nach distal verschoben, zu sehen an dem Spalt zwischen dem Isolatorkörper 1 und dem Rohrabschnitt 50 der Gabelhalterung 5 (Fig. 12).

Dazu ist der Isolatorkörper 1 distalseitig, wie in Fig. 5 bis 10 zu sehen, an einer der beiden voneinander abgewandten Seiten mit einer erste Aufnahmeausnehmung 10 und an der anderen Seite mit einer zweiten Aufnahmeausnehmung 15 geformt. Die Aufnahmeausnehmungen 10, 15 sind jeweils zur schwenkbaren Führung eines Betätigungsabschnitts 20, 20' der Maulteile 2, 2' ausgebildet. Von dem scheibenförmigen Betätigungsabschnitt 20, 20' erstreckt sich der Funktionsabschnitt 23, 23' des jeweiligen Maulteils 2, 2' zum Fassen, Präparieren oder Schneiden und zur Elektrokoagulation.

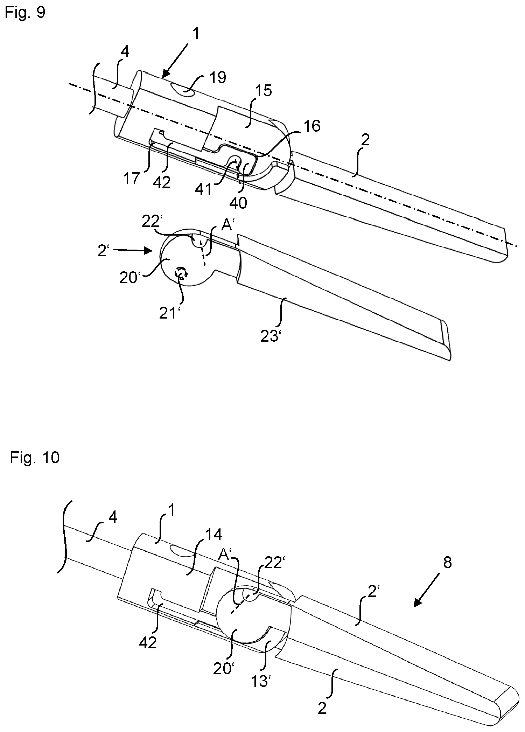

Die Tiefe der Aufnahmeausnehmungen 10, 15 entspricht der Breite des scheibenförmigen Betätigungsabschnitts 20, 20', sodass die Außenseiten der in den Aufnahmeausnehmungen 10, 15 angeordneten Betätigungsabschnitten 20, 20' im Wesentlichen mit den seitlichen Führungsflächen 14 fluchten. Distalseitig werden die Aufnahmeausnehmungen 10, 15 durch einen bezüglich der Längsachse rotationssymmetrischen Führungsabsatz 13, 13' begrenzt. Dieser ist mit einer Viertelkreisbogenbahn zur Führung des scheibenfömigen Betätigungsabschnitts 20, 20' und mit einem zur Längsachse parallelen Anschlag ausgebildet ist, an dem, wie insbesondere in Fig. 10 zu sehen ist, das Maulteil 2, 2' in Schließstellung mit einem Verbindungsabschnitt (unbezeichnet) zwischen Betätigungsabschnitt 20, 20' und Funktionsabschnitt 23, 23' anschlägt.

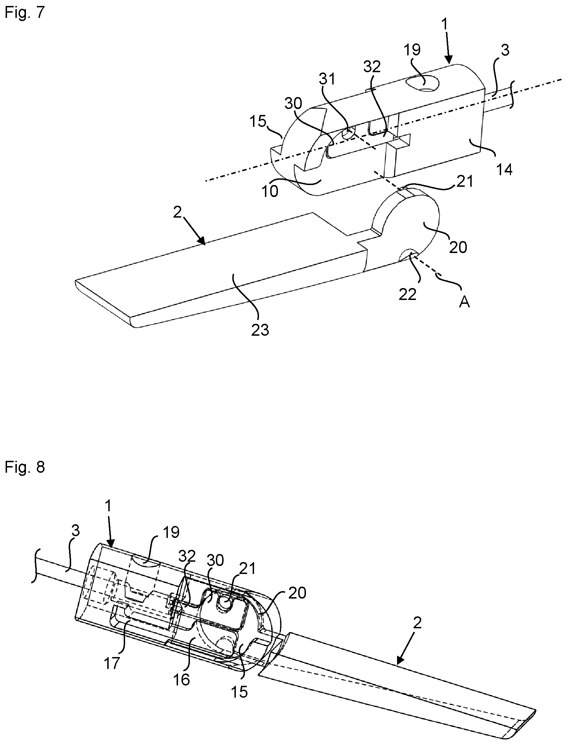

Die vorteilhaft einstückig aus einem elektrisch leitenden Material fertigbaren Maulteile 2, 2' (siehe Fig. 7 bis 10) weisen an dem Betätigungsabschnitt 20, 20' auf einer ersten Seite jeweils einen Betätigungszapfen 21, 21' und auf der davon abgewandten Seite einen Gelenkzapfen 22, 22' auf. Der in Fig. 9 verdeckte Gelenkzapfen 21' des zweiten Maulteils 2' ist gestrichelt dargestellt.

Durch die voneinander beabstandete Anordnung des Betätigungszapfens 21, 21' und des Gelenkzapfens 22, 22' an dem Betätigungsabschnitt 20, 20' jeweils in Randnähe ist der Hebelarm bei der Betätigung der Maulteile möglichst groß. Mit größerem Hebelarm zwischen Betätigungszapfen 21, 21' und Gelenkzapfen 22, 22' kann in der Anwendung das Öffnen und Schließen der Funktionsabschnitte 23, 23' der Maulteile 2, 2' filigraner angesteuert werden. Dann kann bei gleicher Kraft auf das längliche Betätigungsmittel eine höhere Schließkraft zwischen den Funktionsabschnitte 23, 23' der Maulteile 2, 2' erreicht werden.

Daher sind bei einem Maulteilwerkzeug 8 Modifikationen der Betätigungsabschnitte mit den Betätigungs- und Gelenkzapfen bezüglich Form und Abmessungen vom dargestellten Beispiel möglich. Die Gestaltung der Betätigungsabschnitte mit den beiden Zapfen hängt nicht nur von dem zur Verfügung stehenden Bauraum, sondern auch von der Funktion der Maulteile und deren Öffnungswinkel ab, wobei aber meist ein möglichst großer Hebelarm zwischen Betätigungs- und Gelenkzapfen in dem vorhandenen Bauraum angestrebt wird.

Der Gelenkzapfen 22, 22', der die Schwenkachse A, A' des jeweiligen Maulteils 2, 2' definiert, ist in einer an den Gabelarmen 51 vorliegende Schwenköffnung 54 aufgenommen (siehe Fig. 1, 2). Im dargestellten Beispiel ist die Schwenköffnung 54 in einem sichelförmigen Gabelschalenelement 53 ausgebildet, das in einer an den Gabelarmen 51 ausgebildeten Aussparung 52, 52' angeordnet wird und die Montage des Maulteilwerkzeugs 8 erleichtert. Dabei sind die Gabelschalenelemente 53 und die Gabelarme 51 aus einem elektrisch isolierenden Material gefertigt, damit die beiden in den Schwenköffnungen 54 drehbar gelagerten Maulteile 2, 2' nicht durch die Gabelhalterung 5 elektrisch verbunden werden.

Zum mechanischen und elektrischen Eingriff der Maulteile 2, 2' mit dem länglichen Betätigungselement weist der Isolatorkörper 1 in der ersten Aufnahmeausnehmung 10 eine erste Aufnahmetasche 11 auf, die zur Aufnahme eines ersten elektrischen Leitereinsatzes 30 ausgebildet ist (siehe Fig. 5, 7, 8). Analog dazu ist in der zweiten Aufnahmeausnehmung 15 eine zweite Aufnahmetasche 16 zur Aufnahme eines zweiten elektrischen Leitereinsatzes 40 ausgebildet (siehe Fig. 8 bis 10). Die Leitereinsätze 30, 40 werden in ihrer jeweiligen Aufnahmetasche 11, 16 vorzugsweise durch Kleben an dem Isolatorkörper 1 befestigt. Ferner begrenzen die Leitereinsätze 30, 40 jeweils eine Eingriffsöffnung 31, 41, die zur Aufnahme des Betätigungszapfens 21, 21' des jeweiligen Maulteils 2, 2' rotationssymmetrisch in Bezug zur Längsachse L ausgebildet ist. Durch Aufnahme des Betätigungszapfens 21, 21' in der Eingriffsöffnung 31, 41 wird nicht nur der mechanische Eingriff des jeweiligen Maulteils 2, 2' mit dem länglichen Betätigungselement hergestellt, sondern auch die elektrische Kontaktierung über den jeweiligen Leitereinsatz 30, 40. So können über die an dem Isolatorkörper 1 befestigten Leitereinsätze 30, 40 Kraft und Strom von dem Kraftübertragungselement auf die Maulteile 2, 2' übertragen werden.

Die vorliegend U-förmige Eingriffsöffnung 31, 41 in den Leitereinsätzen 30, 40 gestattet ein gewisses Spiel des darin aufgenommenen Betätigungszapfens 21, 21', der sich mit der Schwenkbewegung der Maulteile 2, 2' auf einer Kreisbahn um die jeweilige Schwenkachse A, A' bewegt, während die Eintrittsöffnung 31, 41 mit dem länglichen Betätigungselement linear in Längsrichtung bewegt wird. Dabei bleibt zumindest die Zapfenfläche des Betätigungszapfens 21, 21' immer in tangentialem Kontakt mit dem Leitereinsatz 30, 40 an der Umrandung der Eingriffsöffnung 31, 41. Mit der Scheibenform der Betätigungsabschnitte 20, 20' ist auch ein flächiger Kontakt mit dem jeweiligen Leitereinsatz 30, 40 für einen verringerten elektrischen Übergangs- bzw. Kontaktwiderstand möglich, wenn der Betätigungszapfen 21, 21' des jeweiligen Maulteils 2, 2' in der Eingriffsöffnung 31, 41 des jeweiligen Leitereinsatzes 30, 40 aufgenommen ist.

Zum weiteren Anschluss erstreckt sich von den Leitereinsätzen 30, 40 jeweils ein Leiterabschnitt 32, 42 durch einen Leiterkanal 12, 17. Dieser verbindet die jeweilige Aufnahmetasche 11, 16 mit einer proximalseitigen entlang der Längsachse L ausgebildeten Aufnahmebohrung 18, in die sich das Kraftübertragungselement mit den distalen Enden des inneren Stangen- und äußeren Rohrelements 3, 4 erstreckt. Dabei ragt auch distalseitig das innere Stangenelement 3 mit der inneren Isolierhülle 3' über das äußere Rohrelement 4 mit der äußeren Isolierhülle 4' hinaus, wobei jeweils ein distaler Endabschnitt des inneren Stangenelements 3 und des äußeren Rohrelements 4 unisoliert frei bleibt, um jeweils einen der Leiterabschnitte 32, 42 zu kontaktieren. Dazu sind die Verbindungsstellen der Leiterkanäle 12, 17 mit der Aufnahmebohrung 18 in Bezug auf die Längsachse L voneinander beabstandet und entsprechend die Leiterabschnitte 32, 42 unterschiedlich lang.

Im dargestellten Beispiel ist der erste Leiterkanal 12, der sich von der ersten Aufnahmetasche 11 erstreckt, distalseitig mit der Aufnahmebohrung 18 verbunden, sodass durch den darin angeordneten Leiterabschnitt 32 des ersten Leitereinsatzes 30 das innere Stangenelement 3 kontaktiert wird. Der zweite Leiterkanal 17 mündet an einer proximalen Verbindungsstelle in der Nähe des distalen Endabschnitts des äußeren Rohrelements 4 in die Aufnahmebohrung 18. Der Leiterabschnitt 42 des zweiten Leitereinsatzes 40, der sich durch den zweiten Leiterkanal 17 zur Kontaktierung des äußeren Rohrelements 4 erstreckt, ist daher entsprechend länger als der Leiterabschnitt 32 des ersten Leitereinsatzes 30. Zur Sicherstellung der elektrischen Verbindung sind die Leitereinsätze 30, 40 mit dem Leiterabschnitten 32, 42 einstückig aus einem elektrisch leitenden Material gefertigt, und der Leiterabschnitt 32 des ersten Leitereinsatzes 30 wird mit dem inneren Stangenelement 3 und der Leiterabschnitt 42 des zweiten Leitereinsatzes 40 mit dem äußeren Rohrelement 4 verschweißt.

Zur mechanischen Verbindung des Isolatorkörpers 1 mit dem Kraftübertragungselement wird ein Arretierelement 19' (siehe Fig. 2) verwendet. Das Arretierelement 19' wird in einer Arretieröffnung 19 des Isolatorkörpers 1 angeordnet, die sich radial zu der Aufnahmebohrung 18 erstreckt. Im dargestellten Beispiel liegt die Arretieröffnung 19 nahe distal der Verbindungsstelle des zweiten Leiterkanals 17 mit der Aufnahmebohrung 18 (vgl. Fig. 6). Damit kontaktiert das in der Arretieröffnung 19 angeordnete Arretierelement 19' das Kraftübertragungselement distal zu dem äußeren Rohrelement 4 an dem Kriechstreckenabschnitt, der durch den distalen Endabschnitt der inneren Isolierhülle 3' um das innere Stangenelement 3 gebildet ist, wie in Fig. 2 erkennbar ist.

Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Die vorliegende Erfindung stellt ein bipolares chirurgisches Maulteilwerkzeug 8 für ein ebensolches Instrument 100 bereit, ein bipolares chirurgisches Instrument 100 selbst und ein robotisches chirurgisches Instrumentensystem 110. Dabei weist das Maulteilwerkzeug 8 zwei elektrisch leitfähige, voneinander elektrisch isolierte Maulteile 2, 2', eine Gabelhalterung 5 mit zwei Gabelarmen 51 und ein längliches Betätigungselement auf, das mit den Maulteilen 2, 2' in Eingriff steht. Das Betätigungselement weist distalseitig einen elektrischen Isolatorkörper 1 auf, der zwischen den zwei Gabelarmen 51 geführt wird. Ein erster elektrischer Leitereinsatz 30 und ein zweiter elektrischer Leitereinsatz 40 sind an voneinander abgewandten, den Gabelarmen 51 zugewandten Seiten des Isolatorkörpers 1 befestigt und haben jeweils eine Eingriffsöffnung 31, 41. Jedes Maulteil 2, 2' ist aus einem elektrisch leitenden Material gefertigt und hat an einem Betätigungsabschnitt 20, 20' auf voneinander abgewandten Seiten einen Betätigungszapfen 21, 21' und einen Gelenkzapfen 22, 22', wobei die Gelenkzapfen 22, 22' beider Maulteile 2, 2' in jeweils einer an den Gabelarmen 51 vorliegenden Schwenköffnung 54 aufgenommen sind. Der Betätigungszapfen 21 des ersten Maulteils 2 ist in der Eingriffsöffnung 31 des ersten Leitereinsatzes 30 aufgenommen, und der Betätigungszapfen 21' des zweiten Maulteils 2' ist in der Eingriffsöffnung 41 des zweiten Leitereinsatzes 40 aufgenommen.

### BEZUGSZEICHENLISTE

- 1: Isolatorkörper
- 2, 2': Maulteile
- 3, 3': Inneres Stangenelement, innere Isolierhülle
- 4, 4': Äußeres Rohrelement, äußere Isolierhülle
- 5: Gabelhalterung
- 6: Schaftverbinderhülse
- 7: Instrumentenschaft
- 8: Maulteilwerkzeug
- 9, 9': Betätigungseinheit
- 10: Erste Aufnahmeausnehmung
- 11: Erste Aufnahmetasche
- 12: Leiterkanal
- 13, 13': Führungsabsätze
- 14: Seitliche Führungsfläche
- 15: Zweite Aufnahmeausnehmung
- 16: Zweite Aufnahmetasche
- 17: Leiterkanal
- 18: Aufnahmebohrung
- 19, 19": Arretieröffnung, Arretierbolzen
- 20, 20': Betätigungsabschnitt
- 21, 21': Betätigungszapfen
- 22, 22': Gelenkzapfen
- 23, 23': Funktionsabschnitt
- 30: Erster Leitereinsatz
- 31: Eingriffsöffnung
- 32: Leiterabschnitt
- 39: Proximales Kopplungselement
- 40: Zweiter Leitereinsatz
- 41: Eingriffsöffnung
- 42: Leiterabschnitt
- 50: Rohrabschnitt
- 51: Gabelarm
- 52, 52': Aussparung
- 53: Gabelschalenelement
- 54: Schwenköffnung
- 60: Proximalseitiger Verbindungsabschnitt
- 70: Distalseitiger Verbindungsabschnitt
- 71: Sicherungshülse
- 90: Griffgehäuse
- 91: Bewegliches Griffteil
- 92: Unbewegliches Griffteil
- 93: Bipolaranschluss
- 94: Anschlusselement
- 100: Bipolares chirurgisches Instrument
- 101: Roboterarm
- 102: Steuerungseinheit
- 103: Generator
- 110: Robotisches chirurgisches Instrumentensystem
- A, A': Schwenkachse
- L: Längsachse

## Patentansprüche

1. Bipolares chirurgisches Maulteilwerkzeug (8), das dazu ausgebildet ist, mit einem Instrumentenschaft (7) und mit einer Betätigungseinheit (9) ein bipolares chirurgisches Instrument (100) bereitzustellen, wobei das bipolare Maulteilwerkzeug (8) zwei elektrisch leitfähige, voneinander elektrisch isolierte Maulteile (2, 2'), eine Gabelhalterung (5) mit zwei Gabelarmen (51) und ein längliches Betätigungselement aufweist, wobei die Maulteile (2, 2') zwischen den Gabelarmen (51) um jeweils eine Schwenkachse (A, A') schwenkbar gelagert sind und mit dem Betätigungselement in Eingriff stehen, das entlang seiner Längsachse (L) relativ zur Gabelhalterung (5) beweglich ist,
**dadurch gekennzeichnet, dass**
das längliche Betätigungselement distalseitig einen elektrischen Isolatorkörper (1) aufweist, der zwischen den zwei Gabelarmen (51) geführt wird, wobei ein erster elektrischer Leitereinsatz (30) und ein zweiter elektrischer Leitereinsatz (40) an voneinander abgewandten, den Gabelarmen (51) zugewandten Seiten des Isolatorkörpers (1) befestigt sind und jeweils eine Eingriffsöffnung (31, 41) aufweisen, und jedes Maulteil (2, 2') aus einem elektrisch leitenden Material gefertigt ist und an einem Betätigungsabschnitt (20, 20') auf voneinander abgewandten Seiten einen Betätigungszapfen (21, 21') und einen Gelenkzapfen (22, 22') aufweist, wobei die Gelenkzapfen (22, 22') beider Maulteile (2, 2') in jeweils einer an den Gabelarmen (51) vorliegenden Schwenköffnung (54) aufgenommen sind und die Schwenkachsen (A, A') bereitstellen,
wobei der Betätigungszapfen (21) des ersten Maulteils (2) in der Eingriffsöffnung (31) des ersten Leitereinsatzes (30) und der Betätigungszapfen (21') des zweiten Maulteils (2') in der Eingriffsöffnung (41) des zweiten Leitereinsatzes (40) aufgenommen sind.

2. Maulteilwerkzeug (8) nach Anspruch 1,
**dadurch gekennzeichnet, dass**
das längliche Betätigungselement ein mit dem Isolatorkörper (1) verbundenes Kraftübertragungselement aufweist, das ein elektrisch leitfähiges inneres Stangenelement (3) und ein elektrisch leitfähiges äußeres Rohrelement (4) aufweist, das koaxial um das innere Stangenelement (3) angeordnet und durch eine innere Isolierhülle (3') von dem inneren Stangenelement (3) elektrisch isoliert ist, wobei das innere Stangenelement (3) mit dem ersten Leitereinsatz (30) elektrisch verbunden ist und das äußere Rohrelement (4) mit dem zweiten Leitereinsatz (40) elektrisch verbunden ist.

3. Maulteilwerkzeug (8) nach Anspruch 1 oder 2,
**dadurch gekennzeichnet, dass**
der erste Leitereinsatz (30) und der zweite Leitereinsatz (40) durch einen Klebstoff an dem Isolatorkörper (1) befestigt sind.

4. Maulteilwerkzeug (8) nach Anspruch 1 oder 2,
**dadurch gekennzeichnet, dass**
der erste Leitereinsatz (30) und der zweite Leitereinsatz (40) durch Löten oder Schweißen an dem Isolatorkörper (1) befestigt sind.

5. Maulteilwerkzeug (8) nach zumindest einem der Ansprüche 1 bis 4,
**dadurch gekennzeichnet, dass**
der Isolatorkörper (1) auf den voneinander abgewandten Seiten mit einer ersten Aufnahmeausnehmung (10) und einer zweiten Aufnahmeausnehmung (15) geformt ist, die zur schwenkbaren Führung des Betätigungsabschnitts (20, 20') der Maulteile (2, 2') ausgebildet sind, wobei die erste Aufnahmeausnehmung (10) eine erste Aufnahmetasche (11) aufweist, die zur Aufnahme des ersten Leitereinsatzes (30) ausgebildet ist, und wobei die zweite Aufnahmeausnehmung (15) eine zweite Aufnahmetasche (16) aufweist, die zur Aufnahme des zweiten Leitereinsatzes (40) ausgebildet ist.

6. Maulteilwerkzeug (8) nach zumindest einem der Ansprüche 1 bis 5,
**dadurch gekennzeichnet, dass**
der Isolatorkörper (1) auf den voneinander abgewandten Seiten proximalseitig mit seitlichen Führungsflächen (14) ausgebildet ist, deren Abstand einem Abstand zwischen den Gabelarmen (51) entspricht.

7. Maulteilwerkzeug (8) nach zumindest einem der Ansprüche 2 bis 6,
**dadurch gekennzeichnet, dass**
der Isolatorkörper (1) proximalseitig entlang der Längsachse (L) eine Aufnahmebohrung (18) aufweist, in die sich das Kraftübertragungselement erstreckt, wobei der Isolatorkörper (1) einen ersten Leiterkanal (12) und einen zweiten Leiterkanal (17) aufweist, die sich von der Aufnahmebohrung (18) zu den voneinander abgewandten Seiten des Isolatorkörpers (1) erstrecken, an denen der erste Leitereinsatz (30) und der zweite Leitereinsatz (40) befestigt sind,
wobei der erste Leitereinsatz (30) einen Leiterabschnitt (32) aufweist, der sich durch den ersten Leiterkanal (12) zur Kontaktierung des inneren Stangenelements (3) erstreckt, und der zweite Leitereinsatz (40) einen Leiterabschnitt (42) aufweist, der sich durch den zweiten Leiterkanal (17) zur Kontaktierung des äußeren Rohrelements (4) erstreckt.

8. Maulteilwerkzeug (8) nach Anspruch 7,
**dadurch gekennzeichnet, dass**
eine Verbindungsstelle des ersten Leiterkanals (12) mit der Aufnahmebohrung (18) distal zu einer Verbindungstelle des zweiten Leiterkanals (17) mit der Aufnahmebohrung (18) liegt, und das innere Stangenelement (3) mit der inneren Isolierhülle (3') distal aus dem äußeren Rohrelement (4) hinausragt, wobei die innere Isolierhülle (3') einen distalen Endabschnitt des inneren Stangenelements (3) frei lässt, und ein distaler Endabschnitt der Isolierhülle (3'), der sich distal des äußeren Rohrelements (4) erstreckt, einen Kriechstreckenabschnitt bereitstellt.

9. Maulteilwerkzeug (8) nach Anspruch 7 oder 8,
**dadurch gekennzeichnet, dass**
das Maulteilwerkzeug (8) zur Verbindung des Isolatorkörpers (1) mit dem Kraftübertragungselement ein Arretierelement (19') aufweist, das in einer Arretieröffnung (19) des Isolatorkörpers (1) angeordnet ist, die sich radial zu der Aufnahmebohrung (18) erstreckt.

10. Maulteilwerkzeug (8) nach Anspruch 9,
**dadurch gekennzeichnet, dass**
die Arretieröffnung (19) in einem Bereich der Aufnahmebohrung (18) ausgebildet ist, der sich von der Verbindungsstelle des ersten Leiterkanals (12) zu der Verbindungstelle des zweiten Leiterkanals (17) erstreckt, wobei das in der Arretieröffnung (19) angeordnete Arretierelement (19') das distal aus dem äußeren Rohrelement (4) hinausragende innere Stangenelement (3) mit der inneren Isolierhülle (3') an dem Kriechstreckenabschnitt kontaktiert.

11. Maulteilwerkzeug (8) nach zumindest einem der Ansprüche 1 bis 10,
**dadurch gekennzeichnet, dass**
die Schwenköffnung (54) in einem Gabelschalenelement (53) ausgebildet ist, das in einer an den Gabelarmen (51) ausgebildeten Aussparung (52, 52') anordenbar ist, wobei zumindest die Gabelschalenelemente (53) und / oder die Gabelarme (51) der Gabelhalterung (5) aus einem elektrisch isolierenden Material gefertigt sind.

12. Maulteilwerkzeug (8) nach zumindest einem der Ansprüche 1 bis 11,
**dadurch gekennzeichnet, dass**
jedes Maulteil (2, 2') einteilig aus dem elektrisch leitenden Material gefertigt ist, und / oder
der Betätigungsabschnitt (20, 20') dazu ausgebildet ist, den jeweiligen Leitereinsatz (30, 40) flächig zu kontaktieren, wenn der Betätigungszapfen (21, 21') des jeweiligen Maulteils (2, 2') in der Eingriffsöffnung (31, 41) des jeweiligen Leitereinsatzes (30, 40) aufgenommen ist.

13. Bipolares chirurgisches Instrument (100), das eine Betätigungseinheit (9), einen Instrumentenschaft (7) und ein Maulteilwerkzeug (8) mit zwei in einer Gabelhalterung (5) schwenkbar gelagerten Maulteilen (2, 2') und einem länglichen Betätigungselement aufweist, wobei die elektrisch leitfähigen, voneinander elektrisch isolierten Maulteile (2, 2') mit der Gabelhalterung (5) an einem distalen Ende des Instrumentenschafts (7) angeordnet sind, und sich das längliche Betätigungselement entlang der Längsachse (L) beweglich durch den Instrumentenschaft (7) erstreckt und mit der Betätigungseinheit (9) gekoppelt ist, die an einem proximalen Ende des Instrumentenschafts (7) angeordnet ist,
**dadurch gekennzeichnet, dass**
das Maulteilwerkzeug (8) ein bipolares chirurgisches Maulteilwerkzeug (8) nach zumindest einem der Ansprüche 1 bis 12 ist.

14. Robotisches chirurgisches Instrumentensystem (110), das zumindest eine Steuerungseinheit (102), einen elektrochirurgischen Generator (103) und einen Roboterarm (101) aufweist, der mit der Steuerungseinheit (102) verbunden ist und ein bipolares chirurgisches Instrument (100) aufweist, das mit dem elektrochirurgischen Generator (103) verbunden ist,
**dadurch gekennzeichnet, dass**
das bipolare chirurgische Instrument (100) ein bipolares chirurgisches Instrument (100) nach Anspruch 13 ist.
